# EUROPEAN PATENT SPECIFICATION

(11) **EP 1 965 260 B1**
(45) Date of publication and mention of the grant of the patent: **21.04.2010**
(21) Application number: 08151111.5
(22) Date of filing: 06.02.2008
(51) Int. Cl.: G03G 5/06, C07C 211/54, C07C 211/58

(54) **Imaging member comprising asymmetric arylamine compounds and processes for making these compounds**
Asymmetrische Arylamin-Verbindungen enthaltendes Bilderzeugungselement und Herstellungsverfahren für diese Verbindungen
Élément formant image comprenant des composés assymétriques d'arylamine et procédés de fabrication de ces composés

(30) Priority: 28.02.2007 US 712100
(43) Date of publication of application: 03.09.2008
(73) Proprietor: Xerox Corporation, Rochester, NY 14644 (US)
(72) Inventor: Bender, Timothy P., Toronto Ontario M8Y 1E6 (CA); Coggan, Jennifer A., Cambridge Ontario N3C 4L6 (CA); McGuire, Gregory, Mississauga Ontario L5K 1C7 (CA); Hu, Nan-Xing, Oakville Ontario L6H 7V3 (CA)
(74) Representative: Grünecker, Kinkeldey, Stockmair & Schwanhäusser Anwaltssozietät

(56) References cited:
- EP-A- 0 390 196
- EP-A- 1 850 185
- DE-A- 3 638 418
- JP-A- 63 223 651
- JP-A- 63 271 355
- US-A- 5 837 411
- US-A1- 2002 048 710
- US-A1- 2004 067 427
- US-A1- 2004 126 684
- US-A1- 2006 111 588

## Description

The present disclosure relates generally to the use of compounds useful as hole transport molecules (HTMs) incorporated into imaging members, such as layered photoreceptor devices, and an improved chemical process for making the same. The imaging members can be used in electrophotographic, electrostatographic, xerographic and like devices, including printers, copiers, scanners, facsimiles, and including digital, image-on-image, and like devices. More particularly, the embodiments pertain to the use of asymmetric arylamine compounds used to increase the photoreceptor's "hole mobility," or its ability to move charge, across its charge transport layer (CTL). The embodiments also pertain to an improved chemical process for the synthesis of these arylamine compounds using a tandem Ullmann-Buchwald reaction sequence.

In electrophotography, also known as xerography, electrophotographic imaging or electrostatographic imaging, the surface of an electrophotographic plate, drum, belt or the like (imaging member or photoreceptor) containing a photoconductive insulating layer on a conductive layer is first uniformly electrostatically charged. The imaging member is then exposed to a pattern of activating electromagnetic radiation, such as light. Charge generated by the photoactive pigment moves under the force of the applied field. The movement of the charge through the photoreceptor selectively dissipates the charge on the illuminated areas of the photoconductive insulating layer while leaving behind an electrostatic latent image. This electrostatic latent image may then be developed to form a visible image by depositing oppositely charged particles on the surface of the photoconductive insulating layer. The resulting visible image may then be transferred from the imaging member directly or indirectly (such as by a transfer or other member) to a print substrate, such as transparency or paper. The imaging process may be repeated many times with reusable imaging members.

An electrophotographic imaging member may take one of many different forms. For example, layered photoresponsive imaging members are known in the art. U.S. Pat No. 4,265,990 describes a layered photoreceptor having separate photogenerating and charge transport layers. The photogenerating layer is capable of photogenerating holes and injecting the photogenerated holes into the charge transport layer. Thus, in photoreceptors of this type, the photogenerating material generates electrons and holes when subjected to light.

More advanced photoconductive receptors contain highly specialized component layers. For example, a multilayered photoreceptor that can be employed in electrophotographic imaging systems can include one or more of a substrate, an undercoating layer, an optional hole or charge blocking layer, a charge generating layer (including photogenerating material in a binder, e.g., photoactive pigment) over the undercoating and/or blocking layer, and a charge transport layer (including charge transport material in a binder). Additional layers such as an overcoating layer or layers can also be included. See, for example, U.S. Pat. Nos. 5,891,594 and 5,709,974.

The photogenerating layer utilized in multilayered photoreceptors can include, for example, inorganic photoconductive particles or organic photoconductive particles dispersed in a film forming polymeric binder. Inorganic or organic photoconductive material may be formed as a continuous, homogeneous photogenerating layer.

Upon exposure to light, the charge generated is moved through the photoreceptor. The charge movement is facilitated by the charge transport layer. The speed with which the charge is moved through the charge transport layer directly affects how fast the machine can operate. To achieve the desired increase in machine speed (ppm), the ability of the photoreceptor to move charge must also be increased. Thus, enhancement of charge transport across these layers provides better photoreceptor performance.

Conventional hole transport molecules, such as for example N,N'-diphenyl-N,N-bis(3-methylphenyl)-1,1'-biphenyl-4,4'-diamine, are generally incorporated into the charge transport layer to help mobility. However, the conventional molecules like N,N'-diphenyl-N,N-bis(3-methylphenyl)-1,1'-biphenyl-4,4'-diamine, while providing good mobility, may not impart a high enough mobility for faster machines. As electrophotography advances, there is a growing need to further improve machine speed and devise ways to improve the hole mobility of existing photoreceptors. Thus, there is a need to find new molecules, and ways to synthesize the same, which can allow photoreceptors to obtain faster hole mobility than currently achieved by conventionally used hole transport molecules.

The term "electrostatographic" is generally used interchangeably with the term "electrophotographic." In addition, the terms "charge blocking layer" and "blocking layer" are generally used interchangeably with the phrase "undercoat layer."

US-A-2004/067427 discloses an imaging member comprising a substrate, a charge generating layer, and a charge transporting layer, wherein the charge transporting layer contains an arylamine compound. The arylamine compound may be an asymmetric compound such as N,N'-bis-(4-butyl-phenyl)-N,N'-di-p-tolyl-biphenyl-4,4'-diamine, N,N'-bis-(4-butyl-phenyl)-N,N'-di-m-tolyl-biphenyl-4,4'-diamine, N-(4-butyl-phenyl)-N,N',N'-tri-p-tolyl-biphenyl-4,4'-diamine, N-(4-butyl-phenyl)-N'-phenyl-N'-m-tolyl-N-p-tolyl-biphenyl-4,4'-diamine, N-(4-butyl-phenyl)-N'-phenyl-N,N'-di-m-tolyl-biphenyl-4,4'-diamine, N-(4-butylphenyl)-N-m-tolyl-N',N'-di-p-tolyl-biphenyl-4,4'-diamine, N,N'-bis-(4-butyl-phenyl)-N-m-tolyl-N'-p-tolyl-biphenyl-4,4'-diamine or N-phenyl-N-m-tolyl-N',N'-di-p-tolyl-biphenyl-4,4'-diamine. This publication further discloses a process, which comprises an Ullmann condensation reaction of an diarylamine compound with a diiodide compound.

Imaging members comprising a substrate having provided thereon a layer containing an arylamine compound are also known from US-A-2006/111588, US-A-2004/126684, US-A-2002/048710, DE-A-3638418, JP-A-63-271355, JP-A-63-223651, US-A-5837411, EP-A-390196, and EP-A-1850185.

The present invention provides an imaging member comprising a substrate; a charge generating layer disposed on the substrate; and a charge transporting layer disposed on the charge generating layer, wherein the charge transporting layer comprises a compound selected from N⁴-phenyl-N⁴-biphenyl-N^{4'}-N^{4'}-di-p-tolylbiphenyl-4,4'-diamine, N⁴-phenyl-N⁴-1-naphthalene-N^{4'}-N^{4'}-di-p-tolylbiphenyl-4,4'-diamine, N⁴-phenyl-N⁴-2-naphthalene-N^{4'}-N^{4'}-dip-tolylbiphenyl-4,4'-diamine, and N,N-bis(4-n-butylphenyl)-N',N'-bis(3-methylphenyl)-4,4'-diamino-p-terphenyl, or a mixture thereof.

The present invention further provides a process for forming an asymmetric arylamine compound comprising the steps of (a) condensing a first arylamine with a bromo-iodoaryl or chloro-iodoaryl compound in the presence of a copper catalyst to produce a substituted intermediate; and (b) adding and reacting the substituted intermediate and a second arylamine in the presence of a palladium catalyst. The steps (a) and (b) are preferably performed in tandem and the asymmetric arylamine compound is a tetra(aryl)-biphenyl-diamine-based or tetra(aryl)-terphenyl-diamine-based molecule.
Figure 1 is a cross-sectional view of a multilayered electrophotographic imaging member according to an embodiment of the present disclosure; and
Figure 2 is a schematic nonstructural view showing an embodiment of an electrophotographic image forming apparatus.

The present embodiments relate to the use of compounds useful as hole transport molecules (HTMs) incorporated into imaging members, such as layered photoreceptor devices. These molecules, asymmetric arylamine compounds, are used to increase the photoreceptor's hole mobility across its charge transport layer (CTL), while still providing good coating qualities. Improved chemical processes for making the asymmetric arylamine compounds comprise synthesis using a tandem Ullmann-Buchwald reaction sequence, more specifically, the two reactions are performed sequentially with the Buchwald reaction involving an intermediate produced by the Ullmann reaction. The general process of making arylamines using Buchwald chemistry has been previously described in U.S. Patent Application Publication No. 2006/0111588 to Bender et al.. The embodiments also relate to an imaging member or photoreceptor that incorporates asymmetric arylamine compounds as hole transport molecules to improve hole mobility across the photoreceptor layers.

The asymmetry of the arylamine compounds stems from one end being minimally substituted and another end being substituted with a rigid and/or aromatic group. The minimally substituted end provides more flexibility to the molecule while the other end provides better molecular interaction. This asymmetry provides a hole transport molecule that not only provides good mobility, from the rigid end, but also good coating properties, from the minimally substituted end.

The process comprising an Ullmann condensation reaction and a Buchwald reaction in tandem achieves the formation of two carbon-nitrogen bonds. Buchwald chemistry recognizes the general versatility of palladium-based catalysts for the formation of these bonds. The present disclosure adapts the procedure for the efficient production of asymmetric arylamine compounds. More specifically, as shown below, the Ullmann condensation of an arylamine and a bromo-iodoaryl or cholor-iodoaryl precursor in the presence of a copper catalyst produces a bromide or chloride substituted intermediate, and is followed by a Buchwald reaction of a different arylamine with the bromide or chloride substituted intermediate in the presence of a palladium ligated catalyst. Ullmann reaction: 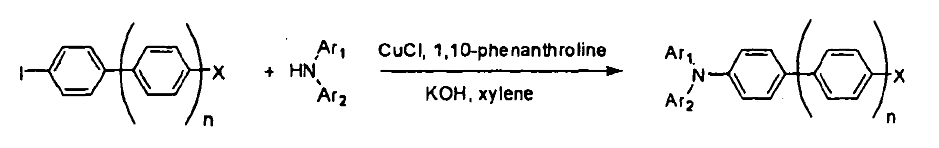 Buchwald reaction:

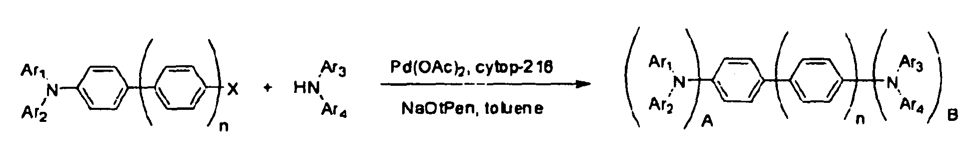

In this reaction scheme, the arylamine can be any suitable arylamine, depending on the desired final product. Thus, for example, in the above reaction scheme, Ar₁ can be any known substituted or unsubstituted aromatic component or a substituted or unsubstituted aryl group having from 2 to 15 conjugate bonded or fused benzene rings and could include, but is not limited to, phenyl, naphthyl, anthryl, phenanthryl, and the like. The substituents on Ar₁, Ar₂, Ar₃, and Ar₄ can be suitably selected to represent hydrogen, a halogen, an alkyl group having from 1 to 20 carbon atoms, a hydrocarbon radical having from 1 to 20 carbon atoms, an aryl group optionally substituted by one or more alkyl groups, an alkyl group containing a heteroatom such as oxygen, nitrogen, sulfur, and the like, having from 1 to 20 carbon atoms, a hydrocarbon radical containing a heteroatom such as oxygen, nitrogen, sulfur, and the like, having from 1 to 20 carbon atoms, an aryl group containing a heteroatom such as oxygen, nitrogen, sulfur, and the like, optionally substituted by one or more alkyl groups, and the like. X represents bromo or chloro, n can be any suitable integer including 0, 1, 2, or 3, and A and B cannot be the same.

In the present embodiments, the intermediate may be, for example, any of the following: N,N-bis(4-methylphenyl)-4-amino-4-bromobiphenyl, N,N-bis(4-methylphenyl)-4-amino-4-chlorobiphenyl, N,N-bis(3,4-dimethylphenyl)-4-amino-4-bromobiphenyl, N,N-bis(3-methylphenyl)-4-amino-4-bromobiphenyl, N,N-bis(4-ethylphenyl)-4-amino-4-chlorobiphenyl N,N-bis(4-n-butylphenyl)-4-amino-4'-bromo-p-terphenyl, N,N-bis(4-methylphenyl)-4-amino-4'-bromo-p-terphenyl, N,N-bis(3,4-dimethylphenyl)-4-amino-4'-chloro-p-terphenyl, N,N-bis(3-methylphenyl)4-amino-4'-chloro-p-terphenyl, N,N-bis(phenyl)-4-amino-4'-bromo-p-terphenyl, and the like. This list of substituted intermediates is not by any means exhaustive.

The tandem synthesis sequence allows selective production of xerographic grade tetra(aryl)-biphenyl-diamine based or tetra(aryl)-terphenyl-diamine-based hole transport molecules for use in photoreceptors. The synthesis process may be conducted in batch mode or in continuous mode, and provides many advantages over the traditional methods of making hole transport molecules. In batch mode, the different samples of starting material are each reacted until a product is obtained before beginning the reaction with the next sample. In continuous mode, the starting materials can be added into the reaction continuously, even after the reaction is initiated, so that the reaction proceeds while the product is being formed. In either mode, the novel tandem synthesis sequence produces the desired asymmetric arylamine compounds in much fewer steps and at lower costs than previously attainable. Moreover, the sequence is able to produce the asymmetric arylamine compounds in much higher, purer yields. In addition, the novel hole transport molecules that are produced by the Ullmann-Buchwald synthesis sequence described herein allow photoreceptors to obtain faster hole mobility than currently achieved by conventionally used hole transport molecules, such as N,N'-diphenyl-N,N-bis(3-methylphenyl)-1,1'-biphenyl-4,4'-diamine. Electrical evaluation on at least one photoreceptor using the presently described asymmetric arylamine compounds was shown to have mobility five times faster than that of a photoreceptor using N,N'-diphenyl-N,N-bis(3-methylphenyl)-1,1'-biphenyl-4,4'-diamine.

According to further embodiments herein, an electrophotographic imaging member is provided, which generally comprises at least a substrate layer, an imaging layer disposed on the substrate, and an optional overcoat layer disposed on the imaging layer. The imaging member includes, as imaging layers, a charge transport layer and a charge generating layer. The imaging member can be employed in the imaging process of electrophotography, where the surface of an electrophotographic plate, drum, belt or the like (imaging member or photoreceptor) containing a photoconductive insulating layer on a conductive layer is first uniformly electrostatically charged. The imaging member is then exposed to a pattern of activating electromagnetic radiation, such as light. The radiation selectively dissipates the charge on the illuminated areas of the photoconductive insulating layer while leaving behind an electrostatic latent image. This electrostatic latent image may then be developed to form a visible image by depositing oppositely charged particles on the surface of the photoconductive insulating layer. The resulting visible image may then be transferred from the imaging member directly or indirectly (such as by a transfer or other member) to a print substrate, such as transparency or paper. The imaging process may be repeated many times with reusable imaging members.

In a typical electrostatographic reproducing apparatus such as electrophotographic imaging system using a photoreceptor, a light image of an original to be copied is recorded in the form of an electrostatic latent image upon an imaging member and the latent image is subsequently rendered visible by the application of a developer mixture. The developer, having toner particles contained therein, is brought into contact with the electrostatic latent image to develop the image on an electrostatographic imaging member which has a charge-retentive surface. The developed toner image can then be transferred to a copy substrate, such as paper, that receives the image via a transfer member.

Alternatively, the developed image can be transferred to another intermediate transfer device, such as a belt or a drum, via the transfer member. The image can then be transferred to the paper by another transfer member. The toner particles may be transfixed or fused by heat and/or pressure to the paper. The final receiving medium is not limited to paper. It can be various substrates such as cloth, conducting or non-conducting sheets of polymer or metals. It can be in various forms, sheets or curved surfaces. After the toner has been transferred to the imaging member, it can then be transfixed by high pressure rollers or fusing component under heat and/or pressure.

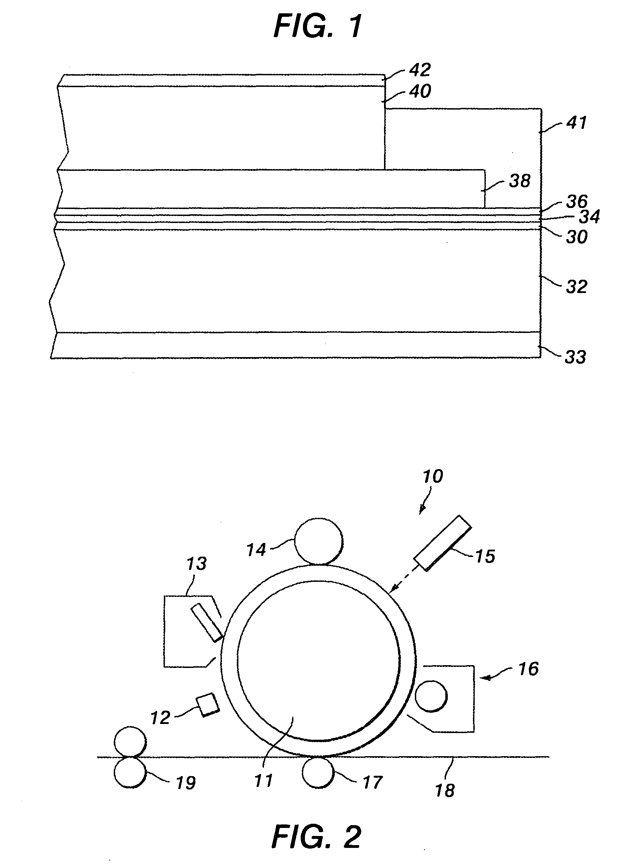

An embodiment of an imaging member is illustrated in Figure 1. The substrate 32 has an optional electrical conductive layer 30. An optional undercoat layer 34 can also be applied over the conductive layer, as well as an optional adhesive layer 36 over the undercoat layer 34. The charge generating layer 38 is illustrated between an adhesive layer 36 and a charge transport layer 40. An optional ground strip layer 41 operatively connects the charge generating layer 38 and the charge transport layer 40 to the conductive layer 30. An anticurl back coating layer 33 may be applied to the side of the substrate 32 opposite from the electrically active layers to render desired imaging member flatness. Other layers of the imaging member may also include, for example, an optional overcoat layer 42 directly over the charge transport layer 40 to provide protection against abrasion and wear.

The conductive ground plane 30 over the substrate 32 is typically a thin, metallic layer, for example a 10 nanometer thick titanium coating, which may be deposited over the substrate by vacuum deposition or sputtering processes. The layers 34, 36, 38, 40 and 42 may be separately and sequentially deposited onto the surface of the conductive ground plane 30 of substrate 32 as wet coating layers of solutions comprising one or more solvents, with each layer being completely dried before deposition of the subsequent coating layer. The anticurl back coating layer 33 may also be solution coated, but is applied to the back side of substrate 32, to balance the curl and render imaging member flashes.

Generally, the thickness of the charge generating layer depends on a number of factors, including the thicknesses of the other layers and the amount of photogenerator material or pigment contained in the charge generating layers. Accordingly, this layer can be of a thickness of, for example, from 0.05 to 5 µm (microns), or from 0.25 to 2 µm (microns) when, for example, the pigments are present in an amount of from 30 to 75 percent by volume. The maximum thickness of this layer in embodiments is dependent primarily upon factors, such as photosensitivity, electrical properties and mechanical considerations. The charge generating layer binder resin present in various suitable amounts, for example from 1 to 50 or from 1 to 10 weight percent, may be selected from a number of known polymers, such as poly(vinyl butyral), poly(vinyl carbazole), polyesters, polycarbonates, poly(vinyl chloride), polyacrylates and methacrylates, copolymers of vinyl chloride and vinyl acetate, phenoxy resins, polyurethanes, poly(vinyl alcohol), polyacrylonitrile, polystyrene, and the like.

Illustrative examples of substrate layers selected for the imaging members may be opaque or substantially transparent, and may comprise any suitable material having the requisite mechanical properties. Thus, the substrate may comprise a layer of insulating material including inorganic or organic polymeric materials, such as MYLAR a commercially available polymer, MYLAR-containing titanium, a layer of an organic or inorganic material having a semiconductive surface layer, such as indium tin oxide, or aluminum arranged thereon, or a conductive material inclusive of aluminum, aluminized polyethylene terephthalate, titanized polyethylene chromium, nickel, brass or the like. The substrate may be flexible, seamless, or rigid, and may have a number of many different configurations, such as for example a plate, a cylindrical drum, a scroll, an endless flexible belt, and the like. In one embodiment, the substrate is in the form of a seamless flexible belt. The anticurl back coating is applied to the back of the substrate.

The thickness of the substrate layer depends on many factors, including economical considerations, thus this layer may be of substantial thickness, for example over 3,000 µm (microns), or of minimum thickness providing there are no significant adverse effects on the member. In embodiments, the thickness of this layer is from 75 to 300 µm (microns).

Moreover, the substrate may contain thereover an undercoat layer in some embodiments, including known undercoat layers, such as suitable phenolic resins, phenolic compounds, mixtures of phenolic resins and phenolic compounds, titanium oxide, silicon oxide mixtures like TiO₂/SiO₂.

In embodiments, the undercoat layer may also contain a binder component. Examples of the binder component include, but are not limited to, polyamides, vinyl chlorides, vinyl acetates, phenolic resins, polyurethanes, aminoplasts, melamine resins, benzoguanamine resins, polyimides, polyethylenes, polypropylenes, polycarbonates, polystyrenes, acrylics, styrene acrylic copolymers, methacrylics, vinylidene chlorides, polyvinyl acetals, epoxys, silicones, vinyl chloride-vinyl acetate copolymers, polyvinyl alcohols, polyesters, polyvinyl butyrals, nitrocelluloses, ethyl celluloses, caseins, gelatins, polyglutamic acids, starches, starch acetates, amino starches, polyacrylic acids, polyacrylamides, zirconium chelate compounds, titanyl chelate compounds, titanyl alkoxide compounds, organic titanyl compounds, silane coupling agents, and combinations thereof. In embodiments, the binder component comprises a member selected from the group consisting of phenolic-formaldehyde resin, melamine-formaldehyde resin, urea-formaldehyde resin, benzoguanamine-formaldehyde resin, glycoluril-fornialdehyde resin, acrylic resin, styrene acrylic copolymer, and mixtures thereof.

In embodiments, the undercoat layer may contain an optional light scattering particle. In various embodiments, the light scattering particle has a refractive index different from the binder and has a number average particle size greater than 0.8 µm. In various embodiments, the light scattering particle is amorphous silica P-100 commercially available from Espirit Chemical Co. In various embodiments, the light scattering particle is present in an amount of 0% to 10% by weight of a total weight of the undercoat layer.

In embodiments, the undercoat layer may contain various colorants. In various embodiments, the undercoat layer may contain organic pigments and organic dyes, including, but not limited to, azo pigments, quinoline pigments, perylene pigments, indigo pigments, thioindigo pigments, bisbenzimidazole pigments, phthalocyanine pigments, quinacridone pigments, quinoline pigments, lake pigments, azo lake pigments, anthraquinone pigments, oxazine pigments, dioxazine pigments, triphenylmethane pigments, azulenium dyes, squarylium dyes, pyrylium dyes, triallylmethane dyes, xanthene dyes, thiazine dyes, and cyanine dyes. In various embodiments, the undercoat layer may include inorganic materials, such as amorphous silicon, amorphous selenium, tellurium, a selenium-tellurium alloy, cadmium sulfide, antimony sulfide, titanium oxide, tin oxide, zinc oxide, and zinc sulfide, and combinations thereof

In embodiments, the thickness of the undercoat layer may be from 0.1 µm to 30 µm.

A photoconductive imaging member herein can comprise in embodiments in sequence of a supporting substrate, an undercoat layer, an adhesive layer, a charge generating layer and a charge transport layer. For example, the adhesive layer can comprise a polyester with, for example, an M_{w} of about 75,000, and an Mₙ of about 40,000.

In embodiments, a photoconductive imaging member further includes an adhesive layer of a polyester with an M_{w} of about 70,000, and an Mₙ of about 35,000. The adhesive layer may comprise any suitable material, for example, any suitable film forming polymer. Typical adhesive layer materials include for example, but are not limited to, copolyester resins, polyarylates, polyurethanes, blends of resins, and the like. Any suitable solvent may be selected in embodiments to form an adhesive layer coating solution. Typical solvents include, but are not limited to, for example, tetrahydrofuran, toluene, hexane, cyclohexane, cyclohexanone, methylene chloride, 1,1,2-trichloroethane, monochlorobenzene, and mixtures thereof, and the like.

In embodiments, the charge transport layer includes a charge transport component and a binder. The charge transport layer may be between 10 µm and 50 µm in thickness. Examples of the binder materials selected for the charge transport layers include components, such as those described in U.S. Patent 3,121,006. Specific examples of polymer binder materials include polycarbonates, polyarylates, acrylate polymers, vinyl polymers, cellulose polymers, polyesters, polysiloxanes, polyamides, polyurethanes, poly(cyclo olefins), and epoxies, and random or alternating copolymers thereof In embodiments electrically inactive binders are comprised of polycarbonate resins with for example a molecular weight of from 20,000 to 100,000 and more specifically with a molecular weight M_{w} of from 50,000 to 100,000. Examples of polycarbonates are poly(4,4'-isopropylidene-diphenylene)carbonate (also referred to as bisphenol-A-polycarbonate, poly(4,4'-cyclohexylidinediphenylene) carbonate (referred to as bisphenol-Z polycarbonate), poly(4,4'-isopropylidene-3,3'-dimethyl-diphenyl)carbonate (also referred to as bisphenol-C-polycarbonate) and the like. In embodiments, the charge transport layer, such as a hole transport layer, may have a thickness from 10 to 55 µm (microns). In embodiments, electrically inactive binders are selected comprised of polycarbonate resins having a molecular weight of from 20,000 to 100,000 or from 50,000 to 100,000. Generally, the transport layer contains from 10 to 75 percent by weight of the charge transport material or from 35 percent to 50 percent of this material.

In embodiments, the at least one charge transport layer comprises from 1 to 7 layers. For example, in embodiments, the at least one charge transport layer comprises a top charge transport layer and a bottom charge transport layer, wherein the bottom layer is situated between the charge generating layer and the top layer.

Figure 2 shows a schematic constitution of an embodiment of an image forming apparatus 10. The image forming apparatus 10 is equipped with an imaging member 11, such as a cylindrical photoreceptor drum, having a charge retentive surface to receive an electrostatic latent image thereon. Around the imaging member 11 may be disposed a static eliminating light source 12 for eliminating residual electrostatic charges on the imaging member 11, an optional cleaning blade 13 for removing the toner remained on the imaging member 11, a charging component 14, such as a charger roll, for charging the imaging member 11, a light-exposure laser optical system 15 for exposing the imaging member 11 based on an image signal, a development component 16 to apply developer material to the charge-retentive surface to create a developed image in the imaging member 11, and a transfer component 17, such as a transfer roll, to transferring a toner image from the imaging member 11 onto a copy substrate 18, such as paper, in this order. Also, the image forming apparatus 10 is equipped with a fusing component 19, such as a fuser/fixing roll, to fuse the toner image transferred onto the copy substrate 18 from the transfer component 17.

The light exposure laser optical system 15 is equipped with a laser diode (for example, oscillation wavelength 780 nm) for irradiating a laser light based on an image signal subjected to a digital treatment, a polygon mirror polarizing the irradiated laser light, and a lens system of moving the laser light at a uniform velocity with a definite size.

### EXAMPLES

The examples set forth herein below are illustrative of different compositions and conditions that can be used in practicing the present embodiments. All proportions are by weight unless otherwise indicated.

### EXAMPLE 1

### Preparation of tetra(aryl)-biphenyl-diamines

### 4-Bromo-4'-iodobiphenyl

Into a 1L 3-neck round bottom flask equipped with mechanical stirrer, thermometer, argon inlet and reflux condenser were placed glacial acetic acid (500 mL), 4-bromobiphenyl (155.2 g), water (76 mL), periodic acid (26.4 g), concentrated sulfuric acid (38 mL) and iodine (72 g). The resulting mixture was heated at 105°C for 2 hours during which time the color of iodine nearly or completely faded. The mixture was cooled to room temperature and filtered. The resulting solid was placed in isopropyl alcohol (500 mL) and heated to a boil, cooled and the resulting solid filtered and washed with 500 mL of methanol. The solid was air dried, then dried under vacuum (80°C/15 mmHg). The resulting yield of 4-bromo-4'-iodobiphenyl was greater than 80% of a white solid. The structure and purity were confirmed by HPLC and 1H NMR.

### N,N-Bis(4-methyphenyl)-4-amino-4-bromobiphenyl

Into a 500mL three necked round bottom flask equipped with a mechanical stirrer, thermometer, argon inlet and reflux condenser were placed di-p-tolylamine (51.7 g), 4-bromo-4-iodobiphenyl (79.9 g), potassium hydroxide (pellets, 88 g), copper(I)oxide (7.5 g), tridecane (25 mL) and toluene (25 mL) which was heated at 180 - 200°C overnight. The mixture was cooled and hydrochloric acid (250 mL) was added and the resulting precipitate stirred for at least 1 hour. The solid was isolated by filtration, collected, dissolved in toluene and treated with a mixture of Filtrol-F-24 and CG-20-Al2O3 while hot (80°C). The absorbents were removed by filtration while hot and the toluene removed by rotary evaporation. The desired compound could be purified by bulb-to-bulb distillation (boiling point about 220°C at 5 mmHg) followed by recrystallization from methanol. The resulting yield of N,N-bis(4-methylphenyl)-4-amino-4-bromobiphenyl was about 50%. The structure and purity were confirmed by HPLC and 1H NMR.

### EXAMPLE 1A

### N⁴-phenyl-N⁴-biphenyl-N^{4'}-N^{4'}-di-p-tolylbiphenyl-4,4'-diamine

120 mg of palladium acetate (Pd(OAc)2, mw 224.51)and 120 mg of Cytop-216 were stirred in toluene (10 mL) for 1 hour at room temperature. To this were added in order 15 mL of aniline (d 1.022, mw 93.13), 12.5 g of 4-bromobiphenyl (mw 233.10), 7 g of sodium t-pentoxide (mw 110.13) and a further 10 mL of toluene. The resulting mixture was heated at 100 - 110°C for between 4 and 6 hours. The mixture was cooled to room temperature and diluted with 20 mL of toluene, filtered to remove insolublcs and 10 - 20 g of Al₂O₃ (CG-20) was added. The resulting slurry was heated to about 80°C and filtered while hot. The toluene was removed by rotary evaporation and the residue was recrystallized from about 150 mL of isopropyl alcohol. The solid produced on cooling was filtered by suction, washed further with about 100 mL of isopropyl alcohol and dried overnight (80°C/15 mmHg). The resulting yield was 8.9 g with a purity of greater than 99%.

Into a 250mL round bottom flask under argon were placed palladium acetate (65 mg) and Cytop-216 (65 mg) which were stirred in toluene (5 mL) at room temperature for 30 minutes. To this were added in order N,N-bis(4-methylphenyl)-4-amino-4-bromobiphenyl (5 g), N-phenyl-4-aminobiphenyl (3.2 g), sodium tert-pentoxide (1.41 g) and toluene (20 mL). The resulting mixture was heated at gentle reflux overnight at which point HPLC analysis confirmed the absence of starting material. The mixture was placed on a rotary evaporator and the toluene removed. The dry powder is mixed with 2 mass equivalents of Al₂O₃ and added to a column already containing 3 mass equivalents of Al₂O₃. Sand is added to the top of the absorbent bed. The column is eluted with refluxing heptane. On cooling, the product precipitates when it was collected and boiled in isopropanol (about 25 mL) for at least 4 hours. The resulting solid was collected by filtration and dried overnight (80°C/15 mmHg). The resulting yield was about 51%.

### EXAMPLE 1B

### N⁴-phenyl-N⁴-1-naphthalene-N^{4'}-N^{4'}-di-p-tolylbiphenyl-4,4'-diamine

Into a 250mL round bottom flask under argon were placed palladium acetate (65 mg) and Cytop-216 (65 mg) which were stirred in toluene (5 mL) at room temperature for 30 minutes. To this were added in order N,N-bis(4-methylphenyl)-4-amino-4-bromobiphenyl (5 g), N-phenyl-1-aminonaphthalene (2.8 g), sodium tert-pentoxide (1.41 g) and toluene (20 mL). The resulting mixture was heated at gentle reflux overnight at which point HPLC analysis confirmed the absence of starting material. The mixture was placed on a rotary evaporator and the toluene removed.. The dry powder is mixed with 2 mass equivalents of Al₂O₃ and added to a column already containing 3 mass equivalents of Al₂O₃. Sand is added to the top of the absorbent bed. The column is eluted with refluxing heptane. On cooling, the product precipitates when it was collected and boiled in isopropanol (about 25 mL) for at least 4 hours. The resulting solid was collected by filtration and dried overnight (80°C/15 mmHg). The resulting yield was about 65%.

### EXAMPLE 1C

### N⁴-henyl-N⁴-2-naphthalene-N^{4'}-N^{4'}-di-p-tolylbiphenyl-4,4'-diamine

Into a 250mL round bottom flask under argon were placed palladium acetate (65 mg) and Cytop-216 (65 mg) which were stirred in toluene (5 mL) at room temperature for 30 minutes. To this were added in order N,N-bis(4-methylphenyl)-4-amino-4-bromobiphenyl (5 g), N-phenyl-2-aminonaphthalene (2.8 g), sodium tert-pentoxide (1.41 g) and toluene (20 mL). The resulting mixture was heated at gentle reflux overnight at which point HPLC analysis confirmed the absence of starting material. The mixture was placed on a rotary evaporator and the toluene removed. The dry powder is mixed with 2 mass equivalents of Al₂O₃ and added to a column already containing 3 mass equivalents of Al₂O₃. Sand is added to the top of the absorbent bed. The column is eluted with refluxing heptane. On cooling, the product precipitates when it was collected and boiled in isopropanol (about 25 mL) for at least 4 hours. The resulting solid was collected by filtration and dried overnight (80°C/15 mmHg). The resulting yield was about 59%.

### EXAMPLE 2

### 4-Bromo-p-terphenyl

Into a 500mL 3-necked round bottom flask equipped with a mechanical stirrer, reflux condenser and drying tube were placed *p*-terphenyl (40g, 173.6mmol), acetic acid (300mL), iodine (30 mg) and bromine (28.8mL, 563.2mmol). The reaction mixture was heated to reflux for 5 hours and after which time the reaction mixture was cooled to room temperature. The white solid precipitate was collected and washed with ethanol to give 41.6g (78%) of 4-bromo-p-terphenyl.

### 4-Bromo-4'-iodo-p-terphenyl

The 4-bromo-p-terphenyl (27 g), iodine (10.53 g), water (20 g), concentrated sulfuric acid (11.5 g), and acetic acid (160 ml) were placed into a 500mL three-necked flask which was heated to 100°C, and stirred by a mechanical stirrer for 20 hours. After cooling to around 60°C, the precipitates were collected by filtration and washed with acetic acid. The solid filtrate was redispersed in isopropanol at 60°C for 30 min, collected by filtration and dried. The pure 4-bromo-4'-iodo-p-terphenyl (23 g) was obtained by sublimation.

### N,N-Bis(4-n-butylphenyl)amine

In a 500mL 3-necked round bottom flask, under argon, equipped with a mechanical stirrer, reflux condenser, thermometer were placed the 1,2,3,4-tetrahydronaphthalene (28mL) and 4-butylaniline (62g, 415.4mmol), followed by addition of calcium chloride (12.8g, 1 15.3mmol) and aluminum chloride (15.4g, 115.49mmol). The resulting mixture was heated at 215°C overnight. The reaction mixture was cooled to room temperature and toluene was added which was then poured into 250mL of water and stirred for 30 minutes. The aqueous layer was removed and the organic layer was washed with 5% aqueous HCl, then saturated aqueous sodium bicarbonate and then water. The organic layer was collected, dried (MgSO4) and concentrated under reduced pressure. The excess 1,2,3,4-tetrahydronaphthalene was removed by Kugelrohr distillation and the di-4-butylphenylamine was used directly in the next step.

### N,N-Bis(4-n-butylphenyl)-4-amino-4'-bromo-p-terphenyl

A mixture of the 4-bromo-4'-iodo-p-tcrphenyl (21.75 g), di-4-butylphenylamine (15.5 g), potassium hydroxide flake (18.0 g), cuprous chloride (0.15), 1,10-phenanthroline monohydrate (0.3 g), and 35 ml of toluene-xylene (V/V: 2:5) were placed in a three-necked flask which was equipped with a Dean-Stark trap and a mechanical stirrer. The mixture was heated to a gentle reflux with efficient stirring under argon for 15 hours. The mixture was cooled to around 60°C, quenched with toluene and water. The organic phase was isolated, and treated with Filtrol-24, followed by alumina. The di-n-butylphenylamine-p-bromoterphenyl was obtained by vacuum distillation.

### N,N-Bis(4-n-butylphenyl)-N',N'-bis(3-methylphenyl)-4-4'-diamino-p-terphenyl

Into a 250mL round bottom flask under argon were placed palladium acetate (11 mg, 0.05mmol), tri-t-butylphosphine (0.01g) and 5mL of toluene. This mixture was stirred for 30 minutes at room temperature. The di-n-butylphenylamine-p-bromoterphenyl (3.0g, 5.1mmol), diphenylamine (1.21 g, 6.1mmol) and sodium t-butoxide (1.5g, 15.3mmol) were added and the reaction mixture was heated to 100°C and was stirred overnight. The reaction mixture was cooled and the toluene was removed under reduced pressure. The residue was placed into 100mL of water and this was stirred for 2 hours. The solid was collected by filtration and was then dissolved into 100mL of toluene and 3g of Filtrol-24 and alumina were added. This mixture was heated to 100°C and then filtered. The toluene was removed under reduced pressure. The dry powder is mixed with 2 mass equivalents of Al₂O₃ and added to a column already containing 3 mass equivalents of Al₂O₃. Sand is added to the top of the absorbent bed. The column is eluted with refluxing heptane. On cooling, the product precipitates and is collected by filtration and vacuum dried overnight to give 2.2g (65%) of the product.

### DEVICE REPARATION

A photoconductor was prepared by providing a 0.02 micrometer thick titanium layer coated (the coater device) on a biaxially oriented polyethylene naphthalate substrate (KALEDEX™ 2000) having a thickness of 89 µm (3.5 mils), and applying thereon, with a gravure applicator, a solution containing 50 grams of 3-amino-propyltricthoxysilane, 41.2 grams of water, 15 grams of acetic acid, 684.8 grams of denatured alcohol, and 200 grams of heptane. This layer was then dried for about 5 minutes at 135°C in the forced air dryer of the coater. The resulting blocking layer had a dry thickness of 500 Angstroms. An adhesive layer was then prepared by applying a wet coating over the blocking layer, using a gravure applicator, and which adhesive contains 0.2 percent by weight based on the total weight of the solution of copolyester adhesive (ARDEL D100™ available from Toyota Hsutsu Inc.) in a 60:30:10 volume ratio mixture of tetrahydrofuran/monochloro-benzene/methylene chloride. The adhesive layer was then dried for about 5 minutes at 135°C in the forced air dryer of the coater. The resulting adhesive layer had a dry thickness of 200 Angstroms.

A photogenerating layer dispersion was prepared by introducing 0.45 grams of the known polycarbonate LUPILON 200™ (PCZ-200) or POLYCARBONATE Z™, weight average molecular weight of 20,000, available from Mitsubishi Gas Chemical Corporation, and 50 milliliters of tetrahydrofuran into a 118 ml (4 ounce) glass bottle. To this solution were added 2.4 grams of hydroxygallium phthalocyanine (Type V) and 300 grams of 1/8-inch (3.2 millimeters) diameter stainless steel shot. This mixture was then placed on a ball mill for 8 hours. Subsequently, 2.25 grams of PCZ-200 were dissolved in 46.1 grams of tetrahydrofuran, and added to the hydroxygallium phthalocyanine dispersion. This slurry was then placed on a shaker for 10 minutes. The resulting dispersion was, thereafter, applied to the above adhesive interface with a Bird applicator to form a photogenerating layer having a wet thickness of 6.4 µm (0.25 mil). A strip about 10 millimeters wide along one edge of the substrate web bearing the blocking layer and the adhesive layer was deliberately left uncoated by any of the photogenerating layer material to facilitate adequate electrical contact by the ground strip layer that was applied later. The charge generation layer was dried at 135°C for 5 minutes in a forced air oven to form a dry photogenerating layer having a thickness of 0.4 micrometer.

The above photogenerating layer was overcoated with a charge transport layer prepared by introducing into an amber glass bottle 50 weight percent of the compound (Examples 1A-1C) to be tested and 50 weight percent of MAKROLON 5705^{®}, a known polycarbonate resin having a molecular weight average of from 50,000 to 100,000, commercially available from Farbenfabriken Bayer A.G. The resulting mixture was then dissolved in methylene chloride to form a solution containing 15 percent by weight solids. This solution was applied on the photogenerating layer to form the bottom layer coating that upon drying (120°C for 1 minute) had a thickness of 30 µm (microns). During this coating process, the humidity was equal to or less than 15 percent.

The above photogenerating layer was overcoated with a charge transport layer prepared by introducing into an amber glass bottle 35 weight percent of the compound (Example 2) to be tested and 65 weight percent of MAKROLON 5705^{®}, a known polycarbonate resin having a molecular weight average of from 50,000 to 100,000, commercially available from Farbenfabriken Bayer A.G. The resulting mixture was then dissolved in methylene chloride to form a solution containing 15 percent by weight solids. This solution was applied on the photogenerating layer to form the bottom layer coating that upon drying (120°C for 1 minute) had a thickness of 30 µm (microns). During this coating process, the humidity was equal to or less than 15 percent.

A control or comparative photoconductor sample was prepared as follows. A metallized mylar substrate was provided and a HOGaPc/poly(bisphenol-Z carbonate) photogenerating layer was machine coated over the substrate. A charge transport layer was then hand coated on the photogenerating layer. The charge transport solution was prepared by mixing 5 grams of N,N'-diphenyl-N,N'-bis(3-methylphenyl)-1,1'-biphenyl-4,4'-diamine (m-TPD), hole transport molecules, and 5 grams of MAKROLON^{®} 5705 in 60 grams of methylene chloride in a bottle by stirring until all solids dissolve. The charge transport layer was coated using a web coating method and by drawing a 12.7 cm (5 inch) 8-path applicator with a 0.25 mm (10 mil) gap across the device to deposit a charge transport layer having a thickness of about 30 µm (micrometers). The coating was dried in a forced air oven for about 1 minute at about 120°C.

### Electrical Property Testing

The above photoreceptors are tested in a scanner set to obtain photoinduced discharge cycles, sequenced at one charge-erase cycle followed by one charge-expose-erase cycle, wherein the light intensity is incrementally increased with cycling to produce a series of photoinduced discharge characteristic curves from which the photosensitivity and surface potentials at various exposure intensities are measured. Additional electrical characteristics are obtained by a series of charge-erase cycles with incrementing surface potential to generate several voltage versus charge density curves. The scanner is equipped with a scorotron set to a constant voltage charging at various surface potentials. The devices are tested at surface potentials of 500 with the exposure light intensity incrementally increased by means of regulating a series of neutral density filters; the exposure light source is a 780 nanometer light emitting diode. The xerographic simulation is completed in an environmentally controlled light tight chamber at ambient conditions (40 percent relative humidity and 22°C).

The xerographic electrical properties of imaging members from examples 1A-C and example 2 were determined by electrostatically charging their surfaces with a corona discharging device, in the dark, until the surface potential attained an initial value V_{ddp} of about 700 volts, as measured 100 ms later by a capacitively coupled probe attached to an electrometer. The charged members were then exposed to light (785 nm, 200 ms after charging) from a filtered xenon lamp. A reduction in the surface potential to V_{bs} background potential due to photodischarge effect, was observed100 ms following exposure. Photodischarge characteristics are represented by E_{1/2}, and E_{7/8} values. E_{1/2} is the exposure energy required to achieve a photodischarge from Vddp to ½ of Vddp and E_{7/8} the energy for a discharge from Vddp to 1 /8 of Vddp. The light energy used to photodischarge the imaging member during the exposure step was measured with a light meter. The higher the photosensitivity, the smaller are E_{1/2} and E_{7/8} values. Residual potential after erase Vr was measured after the device was further subjected to a high intensity white light irradiation from a secondary filtered xenon lamp.

**Table I**

| Sample | HTM loading (%) | E1/2 (ergs/cm²) | E7/8 (ergs/cm²) | Vr (Vots) | Mobility @ 500V | Mobility @ 50V (cm²V-1s-1) |
|---|---|---|---|---|---|---|
| Control m-TPD | 50 | 1.13 | 265 | 15 | 1.37E-05 | 6.31E-08 |
| Example 1A | not soluble | n/a | n/a | n/a | n/a | n/a |
| Example 1B | not soluble | n/a | n/a | n/a | n/a | n/a |
| Example 1C | 50 | 1.21 | 3.57 | 39 | 2.15E-05 | 1.72E-05 |
| Example 2 | 35 | n/a | 3.4 | 38 | 7.73E-08 | 4.67E-08 |
| Control m-TPD | 35 | n/a | 3.30 | 24 | 1.37E-06 | 6.58E-07 |

## Claims

1. An imaging member comprising:
a substrate;
a charge generating layer disposed on the substrate; and
a charge transporting layer disposed on the charge generating layer,
wherein the charge transporting layer comprises a compound selected from N⁴-phenyl-N⁴-biphenyl-N^{4'}-N^{4'}-di-p-tolylbiphenyl-4,4'-diamine, N⁴-Phenyl-N⁴-1-naphthalene-N^{4'}-N^{4'}-di-p-tolylbiphenyl-4,4'-diamine, N⁴-phenyl-N⁴-2-naphthalene-N^{4'}-N^{4'}-di-p-tolylbiphenyl-4,4'-diamine, and N,N-bis(4-n-butylphenyl)-N',N'-bis(3-methylphenyl)-4,4'-diamino-p-terphenyl, or a mixture thereof.

2. A process for forming an asymmetric arylamine compound comprising the steps of:
(a) condensing a first arylamine with a bromo-iodoaryl or chloro-iodoaryl compound in the presence of a copper catalyst to produce a substituted intermediate; and
(b) adding and reacting the substituted intermediate and a second arylamine in the presence of a palladium catalyst.

3. The process of claim 2, wherein steps (a) and (b) are performed in tandem and the asymmetric arylamine compound is a tetra(aryl)-biphenyl-diamine-based or tetra(aryl)-terphenyl-diamine-based molecule.

## Patentansprüche

1. Bilderzeugungselement, umfassend:
ein Substrat;
eine ladungserzeugende Schicht, die auf dem Substrat angeordnet ist; und
eine ladungstransportierende Schicht, die auf der ladungserzeugenden Schicht angeordnet ist,
wobei die ladungstransportierende Schicht eine Verbindung, ausgewählt aus N⁴-Phenyl-N⁴-biphenyl-N^{4'}-N^{4'}-di-p-tolylbiphenyl-4,4'-diamin, N⁴-Phenyl-N⁴-1-naphthalin-N^{4'}-N^{4'}-di-p-tolylbiphenyl-4,4'-diamin, N⁴-Phenyl-N⁴-2-naphthalin-N^{4'}-N^{4'}-di-p-tolylbiphenyl-4,4'-diamin und N,N-Bis(4-n-butylphenyl)-N',N'-bis(3-methylphenyl)-4,4'-diamino-p-terphenyl, oder eine Mischung davon umfasst.

2. Verfahren zum Bilden einer asymmetrischen Arylaminverbindung, umfassend die Schritte:
(a) Kondensieren eines ersten Arylamins mit einer Brom-iodaryl- oder Chlor-iodarylverbindung in Gegenwart eines Kupferkatalysators, um ein substituiertes Zwischenprodukt herzustellen; und
(b) Zugeben und Umsetzen des substituierten Zwischenprodukts und eines zweiten Arylamins in Gegenwart eines Palladiumkatalysators.

3. Verfahren nach Anspruch 2, wobei die Schritte (a) und (b) zusammen durchgeführt werden und die asymmetrische Arylaminverbindung ein Molekül auf Tetra(aryl)-biphenyl-diamin-Basis oder Tetra(aryl)-terphenyl-diamin-Basis ist.

## Revendications

1. Élément d'imagerie comprenant :
un substrat ;
une couche de génération de charge disposée sur le substrat ; et
une couche de transport de charge disposée sur la couche de génération de charge,
dans lequel la couche de transport de charge comprend un composé choisi parmi la N⁴-phényl-N⁴-biphényl-N^{4'}-N^{4'}-di-p-tolylbiphényl-4,4'-diamine, la N⁴-phényl-N⁴-1-naphtalène-N^{4'}-N^{4'}-di-p-tolylbiphényl-4,4'-diamine, la N⁴-phényl-N⁴-2-naphtalène-N^{4'}-N^{4'}-di-p-tolylbiphényl-4,4'-diamine, et le N,N-bis(4-n-butylphényl)-N',N'-bis(3-méthylphényl)-4,4'-diamino-p-terphényle, ou un mélange de ceux-ci.

2. Procédé de formation d'un composé d'arylamine asymétrique comprenant les étapes de :
(a) condensation d'une première arylamine avec un composé bromo-iodoaryle ou chloro-iodoaryle en présence d'un catalyseur à base de cuivre pour produire un intermédiaire substitué ; et
(b) addition et réaction de l'intermédiaire substitué et d'une deuxième arylamine en présence d'un catalyseur à base de palladium.

3. Procédé selon la revendication 2, dans lequel les étapes (a) et (b) sont effectuées en tandem et le composé d'arylamine asymétrique est une molécule à base de tétra(aryl)-biphényl-diamine ou à base de tétra(aryl)-terphényl-diamine.
